# EUROPÄISCHE PATENTSCHRIFT

(11) **EP 2 877 160 B1**
(45) Veröffentlichungstag und Bekanntmachung des Hinweises auf die Patenterteilung: **02.10.2019**
(21) Anmeldenummer: 13747968.9
(22) Anmeldetag: 26.07.2013
(51) Int. Cl.: A61K 9/16, A61K 9/50, A61K 35/02, A61K 33/00

(54) **HEILERDE-ZUBEREITUNG**
MEDICINAL CLAY PREPARATION
PRÉPARATION DE TERRE MÉDICAMENTEUSE

(30) Priorität: 27.07.2012 DE 102012014848
(43) Veröffentlichungstag der Anmeldung: 03.06.2015
(73) Patentinhaber: Heilerde-Gesellschaft Luvos Just GmbH & Co. Kg, 61381 Friedrichsdorf (DE)
(72) Erfinder: KAESTNER, Ariane, 61381 Friedrichsdorf (DE)
(74) Vertreter: Staeger & Sperling Partnerschaftsgesellschaft mbB
(86) Internationale Anmeldenummer: PCT/EP2013/002220
(87) Internationale Veröffentlichungsnummer: WO 2014/015992

(56) Entgegenhaltungen:
- DE-A1-102008 019 339
- JAMINET F: "RESEARCH ON THE ACTION OF ADJUVANTS OF TABLETS ON CERTAIN CHARACTERISTICS OF THE LATTER. I. INFLUENCE OF THE VISCOSITY OF SODIUM CARBOXYMETHYLCELLUSOSES USED AS BINDERS AND AS DISINTEGRATORS ON THE SPEED OF DISINTEGRATION OF KAOLIN TABLETS", JOURNAL DE PHARMACIE DE BELGIQUE, MASSON, PARIS, FR, Bd. 19, 1. März 1964 (1964-03-01), Seiten 144-150, XP009122030, ISSN: 0047-2166
- UEHLEKE B: "Luvos-Heilerde - ein bewährtes Naturheilmittel im Blick neuer Forschung", INTERNET CITATION, Oktober 2005 (2005-10), Seiten 1-4, XP002541877, Gefunden im Internet: URL:http://www.luvos.de/data/luvos/media/d oc/Naturheilkunde_Journal_10_2005.pdf [gefunden am 2005-10-01]

## Beschreibung

Die vorliegende Erfindung betrifft eine Heilerde-Zubereitung mit Hilfsstoffen. Diese Zubereitung enthält mindestens ein Bindemittel und optional ein Überzugsmittel (Coating). Weiterhin betrifft die vorliegende Erfindung ein Verfahren zur Herstellung der Heilerde-Zubereitung. Die Heilerde-Zubereitung hat verbesserte Eigenschaften und kann beispielsweise innerlich angewendet werden.

Heilerde wird aus eiszeitlichen Lössablagerungen gewonnen und ist ein bekanntes und bewährtes Naturheilmittel.

In der Naturmedizin wird Heilerde bzw. Löss zur innerlichen und äußerlichen Anwendung verwendet. Gründe dafür sind die geringe Korngröße, die hohe spezifische Oberfläche und die damit verbundene hohe Ad- und Absorptionsfähigkeit. Auch weist Heilerde ein hohes Säurebindungsvermögen auf.

So wird beispielsweise für die innerliche Anwendung empfohlen, Heilerde bzw. Löss bei Sodbrennen und säurebedingten Magenbeschwerden in Wasser aufgeschlämmt in kleinen Mengen zu schlucken. Hierbei soll die Heilerde den Überschuss an Magensäure und Gallensäuren binden. Dies soll die Magenbeschwerden lindern und den Schutz der Schleimhaut des Magens vor aggressiven Substanzen erhöhen,

Auch Durchfallerkrankungen können mit einer innerlichen Anwendung von Heilerde behandelt werden,

Außerdem können durch die innerliche Anwendung von Heilerde im Darm schädliche Stoffe oder Mikroorganismen gebunden werden, was zur allgemeinen Darmsanierung beiträgt. Eine derartige Darmsanierung ist sowohl krankheitsbedingt als auch prophylaktisch möglich. Hierbei bindet die Heilerde verschiedene Abbauprodukte von Proteinen aus Nahrungseiweiß, wie z.B. Skatol. Weiterhin können die von Darmbakterien ausgeschiedenen Stoffwechselprodukte, die die physiologische Darmflora unter Umständen schädigen, von Heilerde absorbiert bzw. adsorbiert werden.

Äußerlich angewendet lindert Heilerde Akne, Entzündungen, Haut-, Muskel- und Gelenkbeschwerden. Auch in der Kosmetik wird Heilerde zur Haut- und Schönheitspflege angewendet, da sie der Haut ein frisches und schönes Aussehen verleihen soll. Durch die hohe Ad- und Absorptionsfähigkeit der Heilerde erfolgt eine Reinigung der Haut, so dass die Grundlagen für Akne und unreine Haut beseitigt werden.

Zur äußerlichen Anwendung wird die Heilerde meist mit Wasser verrührt und dann beispielsweise als Wickel, Auflage, Bad oder Gesichtsmaske angewendet.

Zur innerlichen Anwendung kann Heilerde beispielsweise in Wasser oder Tee eingerührt eingenommen werden.

Der Rohstoff für Heilerde ist Löss. Dieser wird nach dem Abbau getrocknet, gemahlen und gesiebt. Dadurch liegt die Heilerde als sehr feines Pulver vor. Aufgrund ihrer physischen Beschaffenheit, unter anderem ihrer großen Oberfläche, die neben der stofflich-chemischen Zusammensetzung der Heilerde für die erwünschten und vorteilhaften Wirkungen mitverantwortlich ist, ist eine Weiterverarbeitung des feinvermahlenen Pulvers zu anderen festen Darreichungsformen technologisch schwierig. Reine Heilerde wird daher meist in Pulverform angeboten.

Nur in Ausnahmefällen wird der Löss unvermahlen in groben Brocken oder als grob gebrochenes Granulat in minderer Qualität angeboten, so wie er nach dem Abbau- und Gewinnungsprozeß vorliegt. In dieser Form handelt sich es jedoch um ein nicht standardisiertes Produkt mit schwankenden Eigenschaften.

Ein wichtiger Verarbeitungsschritt ist die Trocknung, damit die Erde rieselfähig wird und weiter verarbeitet werden kann. Außerdem erfolgt während dieses Prozessschritts eine Keimreduktion, so dass die Erde haltbar wird und die erforderlichen maximalen Keimzahlen dauerhaft unterschreitet. Eine rohe, unbearbeitete Erde, bei der noch Aggregate (Heilerde-Brocken) vorhanden sind, sollte nicht an Endanwender weiter gegeben werden. Hochwertige Heilerde wird ausnahmslos in Pulverform oder als Kapseln bzw. Tabletten angeboten.

Heilerde in Pulverform wird in Uehleke, B.: "Luvos-Heilerde - ein bewährtes Naturheilmittel im Blick neuer Forschung", Natur-Heilkunde Journal Medizin Praxis Wissenschaft 10/2005 beschrieben.

Im Fall der innerlichen Anwendung wird bei der Einnahme von Heilerde von den Anwendern teilweise bemerkt, dass die Schluckbarkeit des Heilerde Pulvers nicht optimal ist, da feines Heilerde-Pulver im Mund verbleibt und eine "sandiges" Gefühl bzw. ein Knirschen bemerkbar sind.

Dies lässt sich auch nicht restlos beseitigen, wenn, wie üblicherweise empfohlen, Heilerde in Wasser bzw. Flüssigkeit aufgeschlämmt und dann getrunken wird.

Eine wässrige Heilerde-Suspension ist allerdings optisch nur wenig ansprechend. Der "schlammige Eindruck" des in Wasser eingerührten Pulvers führt aufgrund der Einstellung einiger Menschen, dass man Erde nicht in den Mund nimmt oder isst, zu einer ablehnenden Haltung des Anwenders.

Außerdem sedimentiert die Heilerde im Wasser relativ schnell, was zu einem negativen Geschmackserlebnis für den letzten Rest der Flüssigkeit führt, da in der Neige mehr Heilerde mit weniger Wasser vermischt vorliegt.

Die in Wasser eingerührte Heilerde knirscht überdies im Mund. Auch das trägt dazu bei, dass viele Menschen eine psychologische Barriere bei der Einnahme von Heilerde haben.

Um die Einnahme von in Wasser eingerührter Heilerde mit den beschriebenen Nachteilen zu umgehen, wird Heilerde in Hartgelatinekapseln zum Schlucken angeboten. Es gibt einen Personenkreis, der Kapseln ungern einnimmt oder nicht schlucken kann.

Aufgrund der Ausrichtung und Intention der Naturmedizin, vorzugsweise naturgegebene und reine Stoffe anzuwenden, sollten der Heilerde nach Möglichkeit nicht oder nur möglichst wenig andere Stoffe zugesetzt werden. Weiterhin sollten diese Zusatzstoffe selbst möglichst naturnah, rein oder physiologisch sein, weil damit die unverfälschte Wirkung der Heilerde selbst am besten zum Tragen kommen kann.

In der DE 10 2008 019 339 A1 wird eine Zusammensetzung für die prophylaktische und/oder kurative Behandlung von Hyperazidität beschrieben, insbesondere in Form eines Antazidums, die Heilerde, Alginsäure und eine unter Einwirkung von Säure gasfreisetzende Komponente enthält. Die Zusammensetzung kann in flüssiger oder fester Form, als Suspension, Pulver, Granulat oder Tablette vorliegen. Dabei ist Heilerde in einer Menge von 5 bis 80 Gew.-% und bevorzugt in einer Menge von 20 bis 30 Gew.-% enthalten.

Aufgrund der inhaltstoffe dieser Zusammensetzung mit nur einem relativ geringen Teil Heilerde ist hier eine Granulierung oder Tablettierung der beschriebenen Mischung gut möglich. Für die oben genannte Anwendung im Sinne eines Naturproduktes ist aber eine derartige Kombination von drei verschiedenen Stoffen, nämlich Heilerde, Alginsäure/Alginaten und gasfreisetzender Komponente, nicht erwünscht, da die Heilerde als Naturprodukt möglichst unverfälscht verwendet werden sollte.

In nahezu reiner Form ist Heilerde-Pulver aber bisher kaum zu verarbeiten gewesen, da die raue Beschaffenheit des Pulvers zu hoher Abrasion an den Werkzeugen führt.

Weiterer Stand der Technik im vorliegenden Gebiet ist offenbart in der Veröffentlichung JAMINET F, "RESEARCH ON THE ACTION OF ADJUVANTS OF TABLETS ON CERTAIN CHARACTERISTICS OF THE LATTER. I. INFLUENCE OF THE VISCOSITY OF SODIUM CARBOXYMETHYLCELLUSOSES USED AS BINDERS AND AS DISINTEGRATORS ON THE SPEED OF DISINTEGRATION OF KAOLIN TABLETS", JOURNAL DE PHARMACIE DE BELGIQUE, MASSON, PARIS, FR, (19640301), vol. 19, ISSN 0047-2166, Seiten 144-150.

Es ist daher die Aufgabe der vorliegenden Erfindung, Heilerde in einer Zubereitung zur Verfügung zu stellen, bei der die Anwendung erleichtert ist und die die genannten Nachteile nicht aufweist. Zugleich soll eine Heilerde-Zubereitung gefunden werden, die, insbesondere für die innerliche Anwendung, mit möglichst wenigen Zusatzstoffen versetzt ist.

Weiterhin ist es eine Aufgabe der vorliegenden Erfindung, ein Verfahren zur Verfügung zu stellen, mit welchem die beschriebene erfindungsgemäße Heilerde-Zubereitung hergestellt werden kann.

Die Anmelderin hat nun überraschenderweise herausgefunden, dass sich diese Aufgabe durch eine Heilerde-Zubereitung lösen lässt, die die Merkmale des Anspruchs 1 umfasst. Bevorzugt enthält die Heilerde-Zubereitung 0,5 bis 5,0 Gew.-% Bindemittel und besonders bevorzugt 0,5 bis 2,0 Gew.-% Bindemittel.

Der Begriff "Heilerde" im Sinne der vorliegenden Erfindung umfasst ein aus Lössablagerungen gewonnenes erdiges Tonmineral, Löss ist ein zu den klastischen Gesteinen und hier zu den Silitsteinen gehörendes Sediment. Er besteht aus gleichmäßigem, äußerst feinem, von 8-20 % Kalk-Bruchstücken durchsetztem Quarzstaub mit einem von Eisenhydroxiden gelblich gefärbten tonigen Bindemittel. Der Tonanteil beträgt 5-15 %, der Anteil von Feinsand (klastische Gesteine) 10-20 %.

Mineralogisch besteht Heilerde im Wesentlichen aus Silikaten (über 50 %), Dreischichttonmineralen, Feldspat, Kalzit und Dolomit. Auf Elemente bezogen sind hauptsächlich Sauerstoff, Silizium, Kalzium, Aluminium, Eisen und Kalium; ferner mit Anteilen unter 1 % Magnesium, Natrium, Titan und Phosphor enthalten.

Die Zusammensetzung sowie die Farbe der Heilerde können aufgrund der Tatsache, dass es sich um ein Naturprodukt handelt, sowie aufgrund der Naturgegebenheiten der Abbaustätten variieren.

Heilerde ist ein sehr feinkörniges Pulver. Das Maximum der Korngrößenverteilung bewegt sich zwischen 20 und 50 µm.

Der Ausdruck "enthält" bzw. "enthalten" im Sinne der vorliegenden Erfindung beschreibt eine nicht abschließende Auflistung.

Weiterhin bezieht sich der Begriff "innerliche Anwendung" im Sinne der vorliegenden Anmeldung auf eine Anwendung im Gastro-Intestinal-Trakt und auch im Mund. Unter einer äußerlichen Anwendung wird im Sinne dieser Erfindung die Anwendung auf der Haut, der Kopfhaut und den Haaren verstanden.

Der Begriff "Extrudat" wird für die vorliegende Erfindung weitgehend gleichbedeutend mit dem Begriff "Granulat" verwendet. Gegenstand der vorliegenden Erfindung ist es, aus dem seit langem vorliegenden Heilerde-Pulver eine besser zu handhabende, leicht dosier- und einnehmbare Darreichungsform zu machen. Diese Darreichungsform stellt eine mit Hilfe eines Bindemittels zu größeren Teilchen agglomerierte Heilerde-Zubereitung dar. Diese durch Bindemittel zusammenhaftenden Teilchen werden als Granulat bezeichnet. Das hier beschriebene Granulat kann aufgrund des genannten Herstellungsprozesses (Extrusion) auch als Extrudat bezeichnet werden.

Gemäß einem weiteren Aspekt der Erfindung ist diese Heilerde-Zubereitung enthaltend Heilerde und Bindemittel mit einem Coating in einer Menge von 1,5 bis 5,0 Gew.-% Coating, bezogen auf das Gesamtgewicht der Zubereitung überzogen.

Die Zubereitung liegt vorzugsweise als überzogenes Granulat mit einem mittleren Teilchendurchmesser von etwa 1,5 bis 2,5 mm vor. Besonders geeignet wäre auch ein mittlerer Teilchendurchmesser von etwa 1,8 bis 2,2 mm. In Granulatform hat die Heilerde viele Vorteile gegenüber der bisher bekannten Heilerde in Pulverform.

Die erfindungsgemäße Heilerde-Zubereitung als Granulat ermöglicht dem Anwender eine bequeme Einnahme. So kann das Granulat direkt mit dem Löffel in den Mund gegeben werden. Die Zubereitung ist leicht zu schlucken, entweder indem man das im Mund befindliche Granulat mit Flüssigkeit hinunterschluckt oder indem man es direkt schluckt. Es haftet sich dabei nicht an die Mundschleimhaut an. Weiterhin knirscht die Zubereitung nicht bei der Einnahme und ist überraschenderweise trotz des nur geringen Zusatzes an Bindemittel und optional des Coating-Überzugs geschmacksneutral. Ein nach Anspruch 2 überzogenes Granulat besitzt eine noch leichtere Schluckbarkeit, insbesondere wenn man ohne zusätzliche Flüssigkeit schlucken möchte.

Aufgrund des Bindemittels in der erfindungsgemäßen Konzentration bleibt die Heilerde-Zubereitung nach der Einnahme über einen ausreichenden Zeitraum formstabil bestehen, so dass sie die Mundpassage in der ursprünglichen Form übersteht und sich erst nach dem Schlucken im Magen löst.

Es war nicht zu erwarten, dass bei einer geringen Menge Bindemittel von nur 0,1 bis 10 Gew.-% ein derart stabiles Granulat erhalten werden kann. Zum einen bleibt das Granulat bei einer möglichen Weiterbearbeitung oder Weiterverarbeitung, wie z.B einer Trocknung oder Beschichtung in der Wirbelschicht, trotz des geringen Bindemittel-Anteils stabil. Eine geringe Stabilität des Granulates würde hier zu einem unerwünscht hohen Verlust durch abgeriebene Feinanteile führen. Es kann auch problemlos transportiert, und/oder konfektioniert werden, ohne dass mit einem Zerfall der Agglomerate zu rechnen sein müsste Zum anderen zerfällt es bei der Einnahme im Mund nicht vorzeitig, was wichtig ist für die angenehme Einnahme durch den Anwender. Nur dann nämlich, wenn das Granulat bis nach dem Schlucken stabil bleibt, kann der erdige Geschmack, der bei der Einnahme des Heilerde-Pulvers entsteht, vermieden werden.

Außerdem ist das Granulat aufgrund der besser rieselfähigen Darreichungsform leichter zu handhaben, beispielsweise beim Ausschütten aus dem Vorratsgefäß. Auch eine gleichmäßige Dosierung ist bei einem gut schüttbaren Produkt leichter vorzunehmen.

Gemäß einer weiteren Ausführungsform wird das Granulat portionsweise abgefüllt und kann so direkt aus der Packung in den Mund genommen werden. Auch bei dem Vorgang der portionsweisen Abfüllung hat die Heilerde in Granulat-Form Vorteile gegenüber der Pulver-Form.

Was die erfindungsgemäße Heilerde-Zubereitung anbelangt, so enthält das enthaltene Bindemittel ein oder mehrere wasserlösliche Polymere. Hierbei sind solche Polymere bevorzugt, die möglichst inert sind und/oder aus Naturstoffen bestehen und damit den Prinzipien der Naturheilmittel nicht im Wege stehen. Beispielsweise wären Stärken, Tragant oder Celluloseether denkbar als Bindemittel.

Besonders geeignet sind Celluloseacetat, Ethylcellulose, Methylcellulose Hydroxypropylcellulose, Hydroxypropylmethylcellulose, Carboxymethylcellulose und Polyvinylalkohol.

Vor allem die Celluloseether, wie Methylcellulose oder Hydroxypropylmethylcellulose, sind geeignet. Das Grundgerüst der Celluloseether, das Backbone, ist Cellulose, ein natürlich vorkommendes Polymeres. Durch den teilweisen Ersatz der Hydroxylgruppen wird die Hydrophilie bzw. Hydrophobie den Erfordernissen bestmöglich angepasst. Der Substitionsgrad von Methylcellulose kann bevorzugt bei 1,3 bis 2,6 liegen, insbesondere bei 1,6 bis 2,0; ein bevorzugter Polymerisationsgrad liegt bei etwa 50 bis 1.000. Dies entspricht einer mittleren Molmasse des Methyl-Polymeren von etwa 10.000 bis 220.000 g/Mol.

Da die Celluloseether im Darm nicht abgebaut werden, in kaltem Wasser löslich sind und auch mit den Bestandteilen der Heilerde keine Reaktionen eingehen, sind sie zur Kombination mit diesem Naturprodukt sehr gut geeignet.

Das Coating, mit dem die erfindungsgemäße Heilerde-Zubereitung überzogen ist, enthält ein oder mehrere wasserlösliche Polymere oder Filmbildner. Auch hier ist es zu beachten, dass der Filmbildner möglichst chemisch inert ist und keine unerwünschten Reaktionen mit der Heilerde eingehen kann. Ferner soll der Filmbildner die Wirkung der Heilerde im Körper nicht durch eine eigene Wirkungsweise beeinflussen.

Geeignet sind hier Stärken, modifizierte Stärken, Maltodextrine, Saccharose, Celluloseether, Celluloseacetat.

Diese Filmbildner können insbesondere ausgewählt sein aus Celluloseacetat, Ethylcellulose, methylcellulose, Hydroxypropylmethylcellulose, Polyvinylalkohol, Maltodextrin und Gummi arabicum. Mischungen von zwei oder mehreren der genannten Filmbildner sind ebenfalls möglich.

Besonders bevorzugt sind modifizierte Stärke (z.B. INSTANT PURE-COTE® B793, PURE-DENT® B815) oder Maltodextrine (wie beispielsweise Maltrin® M040, Maltrin® M150, Maltrin® M100).

Die erfindungsgemäß eingesetzte Heilerde zeichnet sich dadurch aus, dass sie aus Löss gewonnen wird, einem eiszeitlichen Sediment. Diese Heilerde enthält Quarzstaub, der zu 8 bis 20 Gew.-% mit kalkigen Bruchstücken durchsetzt ist. Ferner enthält sie einen Anteil von 10 bis 20 Gew.-% Feinsand, der aus klastischen Gesteinen besteht, und von 5 bis 15 Gew.-% an Tonmineral, der durch Eisenhydroxid gelblich gefärbt ist. Die Farbe variiert mit der Zusammensetzung wie es für ein Naturprodukt typisch ist. Der Anteil an Quarzstaub mit kalkigen Bruchstücken ist mit 65 bis 85 Gew.-% am höchsten.

Die erfindungsgemäß eingesetzte Heilerde kann beispielsweise Silikate des Aluminiums, Magnesiums und Kalziums sowie Mischungen dieser Silikate enthalten, weiterhin Dreischichttonminerale, insbesondere Illit, Smectit und/oder Colorit, Feldspaten, Kalzit und Dolomit.

Die genannten Bestandteile der Heilerde weisen mindestens ein Element aus der Gruppe von Silizium, Sauerstoff, Calcium, Aluminium, Eisen, Kalium, Magnesium, Natrium, Titan und Phosphor bevorzugt gebunden als Silikate, Hydroxide oder Carbonate auf.

Außerdem kann die erfindungsgemäß verwendete Heilerde Spurenelemente wie z. B, Kupfer, Mangan, Nickel, Selen und Zink enthalten.

Die Schüttdichte der erfindungsgemäßen Heilerde-Zubereitung kann in einem Bereich von 880 bis 980 g/l liegen. Bevorzugt liegt sie bei 920 bis 940 g/l. Damit liegt sie unter der Schüttdichte von reiner Heilerde.

Die Darreichung als Granulat ist geeignet, größere Einmaldosen als bei den Kapseln zu ermöglichen.

Die Abfüllung der erfindungsgemäßen Heilerde-Zubereitung - beispielsweise als Granulat - kann als Schüttgut in Gebinden von 100 bis 500 g erfolgen. Bevorzugt ist aber eine Abfüllung in Sachets. Hierbei sind Sachets mit einer Einmaldosis von ca. 6 - 7 g besonders vorteilhaft.

Die erfindungsgemäße Heilerde-Zubereitung kann nach einem Verfahren hergestellt werden, bei dem in einem ersten Schritt (a) das Bindemittel in gereinigtem Wasser aufgelöst wird. Vorteilhaft ist das Auflösen des Bindemittels in kaltem gereinigtem Wasser. Anschließend wird in einem zweiten Schritt (b) die Heilerde mit der Bindemittel-Lösung (a) versetzt und gemischt. Dadurch wird die Heilerde befeuchtet. Anschließend wird die feuchte Masse mittels eines gängigen Extruders unter Druck durch Öffnungen definierter Größe gepresst.

Hierbei kann beispielsweise ein Lochmantelextruder, Einschnecken- oder Zweischneckenextruder verwendet werden.

Die Vermischung bzw. Befeuchtung der Heilerde mit der Bindemittellösung kann, je nach Art des verwendeten Extruders, wie oben beschrieben vor dem Eintritt in den Extruder erfolgen oder alternativ im Extruder selbst.

Im Fall, dass die Heilerde erst im Extruder mit Bindemittel-Lösung versetzt wird, ist ein der Extrusion vorgeschalteter Misch- bzw. Befeuchtungsschritt nicht notwendig. Somit ist dieses Verfahren, bei dem die Befeuchtung der Heilerde im Extruder selbst erfolgt, besonders vorteilhaft, da es weniger aufwändig ist und damit auch kostengünstiger.

Das aus der Extrusion gewonnene Heilerde-Granulat wird in einer weiteren Ausführung des erfindungsgemäßen Verfahrens in einer Wirbelschicht getrocknet.

Anschließend kann das Granulat optional in einem Schritt (c) mit einem Coating nach einem üblichen Verfahren beschichtet werden.

Besonders bevorzugt geschieht diese Beschichtung in einem direkt an die Trocknung anschließenden Arbeitsgang in derselben Wirbelschicht oder es wird gleichzeitig getrocknet und beschichtet.

Sowohl die Trocknung als auch die optionale Beschichtung nach (c) können solange fortgeführt werden, bis die gewünschte Endfeuchte erreicht ist und die Beschichtung in der gewünschten Stärke aufgebracht ist.

Trotz des niedrigen Gehalts an Bindemittel von 0,1 bis 10 Gew.-% erhält man ein Extrudat, das ausreichende Stabilität aufweist.

Die vorliegende Erfindung wird anhand der nachfolgenden Ausführungsbeispiele erläutert, die lediglich der Veranschaulichung dienen ohne die Erfindung auf den Umfang dieser Beispiele zu beschränken.

Zusammensetzungen 1, 3, 5 und 7 fallen nicht unter die Ansprüche.

### Zubereitung 1

Aus folgenden Inhaltsstoffen wird eine Zubereitung in Form eines Granulates hergestellt:

| In haltsstoffe | Menge in kg pro Charge | Menge in Gew.-% im Endprodukt |
|---|---|---|
| Heilerde | 400,0 | 99,09 |
| Methylcellulose | 3,68 | 0,91 |
| Wasser (gereinigt) | q.s. | |

Die erforderliche Menge an Methylcellulose wird in einer definierten Menge kaltem Wasser gelöst.

Zur Granulation wird der Löss mit Methylcelluloselösung befeuchtet und gebunden. Der so befeuchtete Löss wird nach dem Einbringen der gesamten vorgesehenen Bindemittellösung zum Einzug des Einschneckenextruders befördert. Hierzu verwendet man einen Silikonschlauch, über den die feuchte Mischung direkt in den Materialeinlass des Extruders gelangt. Die Extrusion erfolgt durch eine 2,0 mm Matrize.

Anschließend an die Extrusion wird das noch feuchte Granulat in einer Wirbelschichtanlage getrocknet. Während des Trocknungsschrittes erfolgt eine mäßige Erhitzung des Produktes auf 25 bis 35 °C.

### Zubereitung 2 (erfindungsgemäß)

| Inhaltsstoffe | Menge in kg pro Charge | Menge in Gew.-% im Endprodukt |
|---|---|---|
| Heilerde-Methylcellulose-Granulat | 390,60 | 97,65 |
| Methylcellulose | 6,28 | 1,57 |
| Maltodextrin | 3,12 | 0,78 |
| Wasser | q.s. | |

Maltodextrin und Methylcellulose werden locker vermischt und in der erforderlichen Menge kaltem gereinigtem Wasser gelöst.

Das als Zubereitung 1 erhaltene Extrudat wird in der Wirbelschichtanlage mit der Methylcellulose-Maltodextrin-Lösung nach und nach besprüht, bis die gesamte Menge an Beschichtungslösung (Coating) aufgebracht ist. Anschließend wird die Wirbelschicht noch so lange bei ähnlicher Temperatur weiterbetrieben, bis auch das Coating getrocknet ist.

Nach Aufbringen der erforderlichen Menge an Coating und unterschreiten der maximalen Restfeuchte wird das Granulat gekühlt. Das fertige Produkt wird aus dem Wirbelbett ausgetragen und einer Schutzsiebung unterzogen. Dabei werden sowohl Überkorn (> 2,5 mm) wie auch Unterkorn (< 1 mm) abgesiebt.

Das fertige Zwischenprodukt wird zur Zwischenlagerung und zum Weitertransport als 20 kg Gebinde in PE-Beutel abgefüllt und fest verschlossen.

### Zubereitung 3

Aus folgenden Inhaltsstoffen wird eine Zubereitung in Form eines Granulates hergestellt:

| Inhaltsstoffe | Menge in kg pro Charge | Menge in Gew.-% im Endprodukt |
|---|---|---|
| Heilerde | 392,20 | 98,05 |
| Ethylcellulose | 4,20 | 1,05 |
| Methylcellulose | 3,60 | 0,90 |
| Wasser (gereinigt) | q.s. | |

Die erforderliche Menge an Ethylcellulose und Methylcellulose werden locker vermischt und in einer ausreichenden Menge kaltem Wasser gelöst.

Zur Granulation im Zweischneckenextruder wird die Heilerde direkt in die Einzugszone des Extruders gegeben. In der Verdichtungszone des Extruders wird sie während des Verdichtens mit der Ethylcellulose-Methylcelluloselösung befeuchtet. Ein der Granulation vorgeschalteter Mischschritt von Heilerde und Bindemittel-Lösung ist nicht notwendig. Die Extrusion erfolgt durch eine 2,0 mm Matrize.

Anschließend an die Extrusion wird das noch feuchte Granulat in einer Wirbelschichtanlage getrocknet. Während des Trocknungsschrittes erfolgt eine mäßige Erhitzung des Produktes auf 25 bis 35 °C.

### Zubereitung 4 (erfindungsgemäß)

Das unter 3 erhaltene Granulat wird mit folgenden Zusatzstoffen überzogen:

| Inhaltsstoffe | Menge in kg pro Charge | Menge in Gew.-% im Endprodukt |
|---|---|---|
| Heilerde-Ethylcelloluse-Methylcellulose-Granulat | 387,0 | 96,75 |
| Ethylcellulose | 10,0 | 2,50 |
| Methylcellulose | 1,6 | 0,40 |
| Maltodextrin | 1,6 | 0,40 |
| Wasser | q.s. | |

Ethylcellulose, Methylcellulose und Maltodextrin werden locker vermischt und in der erforderlichen Menge kaltem gereinigtem Wasser gelöst.

Das als Zubereitung 3 erhaltene Extrudat wird- in der Wirbelschichtanlage mit der Ethylcellulose-Methylcellulose-Maltodextrin-Lösung nach und nach besprüht, bis die gesamte Menge an Beschichtungslösung (Coating) aufgebracht ist. Anschließend wird die Wirbelschicht noch so lange bei ähnlicher Temperatur weiterbetrieben, bis auch das Coating getrocknet ist.

Nach Aufbringen der erforderlichen Menge an Coating und unterschreiten der maximalen Restfeuchte wird das Granulat gekühlt. Das fertige Produkt wird aus dem Wirbelbett ausgetragen und einer Schutzsiebung unterzogen. Dabei werden sowohl Überkorn (> 2,5 mm) wie auch Unterkorn (< 1 mm) abgesiebt.

Das fertige Zwischenprodukt wird zur Zwischenlagerung und zum Weitertransport als 20 kg Gebinde in PE-Beutel abgefüllt und fest verschlossen.

### Zubereitung 5

Aus folgenden Inhaltsstoffen wird eine Zubereitung in Form eines Granulates hergestellt:

| Inhaltsstoffe | Menge in kg pro Charge | Menge in Gew.-% im Endprodukt |
|---|---|---|
| Heilerde | 396,0 | 99,0 |
| Hydroxypropyl-Methylcellulose | 4,0 | 1,0 |
| Wasser (gereinigt) | q.s. | |

Die erforderliche Menge an Hydroxypropylmethylcellulose wird in einer ausreichenden Menge kaltem Wasser gelöst.

Zur Granulation im Zweischneckenextruder wird die Heilerde direkt in die Einzugszone des Extruders gegeben. In der Verdichtungszone des Extruders wird sie während des Verdichtens mit der Hydroxypropylmethylcelluloselösung befeuchtet. Ein der Granulation vorgeschalteter Mischschritt von Heilerde und Bindemittel-Lösung ist nicht notwendig. Die Extrusion erfolgt durch eine 2,0 mm Matrize.

Anschließend an die Extrusion wird das noch feuchte Granulat in einer Wirbelschichtanlage getrocknet. Während des Trocknungsschrittes erfolgt eine mäßige Erhitzung des Produktes auf 25 bis 35 °C.

### Zubereitung 6 (erfindungsgemäß)

Das unter 5 erhaltene Granulat wird mit folgenden Zusatzstoffen überzogen:

| Inhaltsstoffe | Menge in kg pro Charge | Menge in Gew.-% im Endprodukt |
|---|---|---|
| Heilerde-HPMC-Granulat | 389,8 | 97,45 |
| Hydroxypropyl-Methylcellulose | 6,72 | 1,67 |
| Maltodextrin | 3,56 | 0,88 |
| Wasser | q.s. | |

Hydroxypropylmethylcellulose und Maltodextrin werden locker vermischt und in der erforderlichen Menge kaltem gereinigtem Wasser gelöst.

Das als Zubereitung 5 erhaltene Extrudat wird in der Wirbelschichtanlage mit der Hydroxypropylmethylcellulose-Maltodextrin-Lösung nach und nach besprüht, bis die gesamte Menge an Beschichtungslösung (Coating) aufgebracht ist. Anschließend wird die Wirbelschicht noch so lange bei ähnlicher Temperatur weiterbetrieben, bis auch das Coating getrocknet ist.

Nach Aufbringen der erforderlichen Menge an Coating und unterschreiten der maximalen Restfeuchte wird das Granulat gekühlt. Das fertige Produkt wird aus dem Wirbelbett ausgetragen und einer Schutzsiebung unterzogen. Dabei werden sowohl Überkorn (> 2,5 mm) wie auch Unterkorn (< 1 mm) abgesiebt.

Das fertige Zwischenprodukt wird zur Zwischenlagerung und zum Weitertransport als 20 kg Gebinde in PE-Beutel abgefüllt und fest verschlossen.

### Zubereitung 7

Aus folgenden Inhaltsstoffen wird eine Zubereitung in Form eines Granulates hergestellt:

| Inhaltsstoffe | Menge in kg pro Charge | Menge in Gew.-% im Endprodukt |
|---|---|---|
| Heilerde | 399,84 | 99,96 |
| Ethylcellulose | 0,12 | 0,03 |
| Methylcellulose | 0,04 | 0,01 |
| Wasser (gereinigt) | q.s. | |

Ethylcellulose und Methylcellulose werden locker gemischt und in der erforderlichen Menge kaltem gereinigtem Wasser gelöst.

Die mit der Bindemittellösung befeuchtete Heilerde wird in einen Einschneckenextruder eingebracht. Die extrudierte Masse zerfällt zu einem Teil bereits beim Einbringen in die Wirbelschicht. Nach kurzer Zeit in der Wirbelschicht sind keine brauchbaren Agglomerate mehr vorhanden.

Somit ist gezeigt, dass die in der vorliegenden Erfindung verwendeten Mengen an Heilerde und Bindemittel derart gewählt sind, dass auch mit einer relativ geringen Menge an Bindemittel ein stabiles Granulat erhalten wird.

Dieses Granulat hat die beschriebenen Vorteile gegenüber dem bisher verwendeten Heilerde-Pulver ohne dass unnötig viel Hilfsstoff eingesetzt wäre.

## Patentansprüche

1. Heilerde-Zubereitung, enthaltend 90 - 99,9 Gew.-% Heilerde und 0,1 - 10 Gew.-% Bindemittel,
wobei das Bindemittel ein oder mehrere wasserlösliche Polymere enthält,
wobei jedes wasserlösliche Polymer des Bindemittels ausgewählt ist aus der Gruppe enthaltend Stärken, Tragant oder Celluloseacetat, Celluloseether, Ethylcellulose, Methylcellulose Hydroxypropylcellulose, Hydroxypropylmethylcellulose, Carboxymethylcellulose und Polyvinylalkohol,
wobei die Heilerde-Zubereitung überzogen ist mit einem Coating in einer Menge von 1,5 bis 5,0 Gew.-%, bezogen auf das Gesamtgewicht der überzogenen Zubereitung,
wobei das Coating einen oder mehrere wasserlösliche Polymere oder Filmbildner enthält, und wobei jeder wasserlösliche Filmbildner oder jedes wasserlösliche Polymer des Coatings ausgewählt ist aus der Gruppe enthaltend Stärke, modifizierte Stärke, Saccharose, Celloluseacetat, Ethylcellulose, Methylcellulose, Hydroxypropylmethylcellulose, Polyvinylalkohol, Maltodextrin, Gummi arabicum
wobei die Zubereitung als überzogenes Granulat vorliegt.

2. Heilerde-Zubereitung nach Anspruch 1, enthaltend 95 - 99,5 Gew.-% Heilerde und 0,5 - 5,0 Gew.-% Bindemittel.

3. Heilerde-Zubereitung nach Anspruch 1, enthaltend 98 - 99,5 Gew.-% Heilerde und 0,5 - 2,0 Gew.-% Bindemittel.

4. Heilerde-Zubereitung nach einem der Ansprüche 1 bis 3,
wobei die Heilerde ein Löss ist mit einem Anteil an Quarzstaub von 65 bis 85 Gew.-%, der von 8 bis 20 Gew.-% kalkigen Bruchstücken durchsetzt ist, einem Anteil an Tonmineral von 5 bis 15 Gew.-% und einem Anteil von klastischen Gesteinen von 10 bis 20 Gew.-%.

5. Heilerde-Zubereitung nach einem der Ansprüche 1 bis 3,
wobei die Heilerde einen oder mehrere Bestandteile aus der Gruppe von Silikaten des Aluminiums, Magnesiums und Kalziums sowie Mischungen dieser Silikate, Dreischichttonmineralen, insbesondere Illit, Smectit und/oder Colorit, Feldspaten, Kalzit und Dolomit enthält.

6. Heilerde-Zubereitung nach einem der Ansprüche 1 bis 3,
wobei die Heilerde mindestens ein Element aus der Gruppe von Silizium, Sauerstoff, Calcium, Aluminium, Eisen, Kalium, Magnesium, Natrium, Titan und Phosphor enthält und/oder
wobei in der Heilerde als Spurenelement mindestens eines der Elemente Kupfer, Mangan, Nickel, Selen und/oder Zink enthalten ist.

7. Heilerde-Zubereitung nach einem der Ansprüche 1 bis 4,
wobei die Zubereitung eine Schüttdichte von 880 bis 980 g/l, bevorzugt eine Schüttdichte von 920 bis 940 g/l aufweist.

8. Verfahren zur Herstellung einer Heilerde-Zubereitung nach einem der Ansprüche 1 bis 7, wobei
(a) das bzw. die Bindemittel in gereinigtem Wasser aufgelöst bzw. suspendiert werden,
(b) die Heilerde mit dem Bindemittel-Wasser-Gemisch nach (a) versetzt wird und nach einem üblichen Verfahren granuliert wird, und
(c) der oder die wasserlöslichen Polymere oder Filmbildner des Coatings in gereinigtem Wasser aufgelöst oder suspendiert werden, und
(d) das resultierende Granulat nach (b) mit dem Coating-Wasser-Gemisch nach (c) nach einem üblichen Verfahren beschichtet wird.

9. Verfahren nach Anspruch 8,
wobei der Verfahrensschritt (b) mit einem Extruder durchgeführt wird.

10. Verfahren nach Anspruch 8 oder 9, wobei der Verfahrensschritt nach (b) und/oder der Verfahrensschritt nach (d) in einer Wirbelschicht durchgeführt wird.

11. Verfahren nach Anspruch 10,
wobei der Verfahrensschritt nach (b) und/oder Verfahrensschritt nach (d) solange durchgeführt wird, bis die gewünschte Coating-Stärke und die erforderliche Restfeuchte erreicht sind.

## Claims

1. Healing earth preparation containing 90 - 99.9 % by weight of healing earth and 0.1 - 10 % by weight of binder,
wherein the binder comprises one or more water-soluble polymers,
wherein each water-soluble polymer of the binder is selected from the group consisting of starch, tragacanth or cellulose acetate, cellulose ether, ethyl cellulose, methyl cellulose, hydroxypropyl cellulose, hydroxypropyl methyl cellulose, carboxymethyl cellulose and polyvinyl alcohol,
wherein the healing earth preparation is coated with a coating in an amount of 1.5 to 5.0 % by weight based on the total weight of the coated preparation,
wherein the coating comprises one or more water soluble polymers or film formers, and wherein each water soluble film former or soluble polymer of the coating is selected from the group consisting of starch, modified starch, sucrose, cellulose acetate, ethylcellulose, methylcellulose, hydroxypropylmethylcellulose, polyvinyl alcohol, maltodextrin, gum arabic the preparation being in the form of coated granules.

2. Healing earth preparation according to claim 1, containing 95 - 99.5 % by weight of healing earth and 0.5 - 5.0 % by weight of binder.

3. Healing earth preparation according to claim 1, containing 98 - 99.5 % by weight of healing earth and 0.5 - 2.0 % by weight of binder.

4. Healing earth preparation according to one of the claims 1 to 3,
wherein the healing earth is a loess having a proportion of quartz dust of 65 to 85 % by weight interspersed with 8 to 20 % by weight of calcareous fragments, a proportion of clay mineral of 5 to 15 % by weight and a proportion of clastic rocks of 10 to 20 % by weight.

5. Healing earth preparation according to one of the claims 1 to 3,
wherein the healing earth contains one or more components from the group of silicates of aluminium, magnesium and calcium as well as mixtures of these silicates, three-layer clay minerals, in particular illite, smectite and/or colorite, feldspars, calcite and dolomite.

6. Healing earth preparation according to one of the claims 1 to 3,
wherein the healing earth contains at least one element from the group consisting of silicon, oxygen, calcium, aluminium, iron, potassium, magnesium, sodium, titanium and phosphorus, and/or
at least one of the elements copper, manganese, nickel, selenium and/or zinc being present in the healing earth as trace element.

7. Healing earth preparation according to one of the claims 1 to 4,
wherein the preparation has a bulk density of 880 to 980 g/l, preferably a bulk density of 920 to 940 g/l.

8. Method for preparing a healing earth preparation according to any of the claims 1 to 7, wherein
(a) the binder(s) are dissolved or suspended in purified water,
(b) the healing earth is mixed with the binder-water mixture according to (a) and granulated according to a conventional process, and
(c) the water soluble polymer(s) or film former(s) of the coating are dissolved or suspended in purified water; and
(d) the resulting granules of (b) are coated with the coating water mixture of (c) by a conventional process.

9. Method according to claim 8,
wherein method step (b) is executed with an extruder.

10. Method according to claim 8 or 9, wherein the method step according to (b) and/or the method step according to (d) is executed in a fluidized bed.

11. Method according to claim 10,
wherein the method step according to (b) and/or method step according to (d) is executed until the desired coating strength and the required residual moisture are achieved.

## Revendications

1. Préparation de terre médicamenteuse contenant 90-99,9 % en poids de terre médicamenteuse et 0,1-10 % en poids d'agent liant,
dans laquelle l'agent liant contient un ou plusieurs polymères hydrosolubles,
dans laquelle chaque polymère hydrosoluble de l'agent liant est choisi dans le groupe contenant des amidons, de la gomme adragante ou de l'acétate de cellulose, de l'éther de cellulose, de l'éthylcellulose, de la méthylcellulose, de l'hydroxypropylcellulose, de l'hydroxypropylméthyl-cellulose, de la carboxyméthylcellulose et de l'alcool polyvinylique,
dans laquelle la préparation de terre médicamenteuse est recouverte d'un revêtement en quantité de 1,5 à 5,0 % en poids par rapport au poids total de la préparation recouverte,
dans laquelle le revêtement contient un ou plusieurs polymères ou agents filmogènes hydrosolubles et dans laquelle chaque agent filmogène hydrosoluble ou chaque polymère hydrosoluble du revêtement est choisi dans le groupe contenant des amidons, des amidons modifiés, du saccharose, de l'acétate de cellulose, de l'éthylcellulose, de la méthylcellulose, de l'hydroxypropylméthylcellulose, de l'alcool polyvinylique, de la maltodextrine et de la gomme arabique,
dans laquelle la préparation se présente sous la forme d'un granulé recouvert.

2. Préparation de terre médicamenteuse selon la revendication 1, contenant 95-99,5 % en poids de terre médicamenteuse et 0,5-5,0 % en poids d'agent liant.

3. Préparation de terre médicamenteuse selon la revendication 1, contenant 98-99,5 % en poids de terre médicamenteuse et 0,5-2,0 % en poids d'agent liant.

4. Préparation de terre médicamenteuse selon l'une quelconque des revendications 1 à 3,
dans laquelle la terre médicamenteuse est un loess avec une fraction de poudre de quartz de 65 à 85 % en poids, qui est chargée de 8 à 20 % en poids de fragments calcaires, une fraction de minéral argileux de 5 à 15 % en poids et une fraction de roches clastiques de 10 à 20 % en poids.

5. Préparation de terre médicamenteuse selon l'une quelconque des revendications 1 à 3,
dans laquelle la terre médicamenteuse contient un ou plusieurs composants choisis dans le groupe des silicates d'aluminium, de magnésium et de calcium ainsi que des mélanges de ces silicates, des minéraux argileux à trois couches, en particulier de l'illite, de la smectite et/ou de la colorite, des feldspaths, de la calcite et de la dolomite.

6. Préparation de terre médicamenteuse selon l'une quelconque des revendications 1 à 3,
dans laquelle la terre médicamenteuse contient un élément choisi dans le groupe du silicium, de l'oxygène, du calcium, de l'aluminium, du fer, du potassium, du magnésium, du sodium, du titane et du phosphore et/ou
dans laquelle au moins un des éléments cuivre, manganèse, nickel, sélénium et/ou zinc est contenu dans la terre médicamenteuse comme oligo-élément.

7. Préparation de terre médicamenteuse selon l'une quelconque des revendications 1 à 4,
dans laquelle la préparation présente une masse spécifique apparente de 880 à 980 g/l, de préférence une masse spécifique apparente de 920 à 940 g/l.

8. Procédé de fabrication d'une préparation de terre médicamenteuse selon l'une quelconque des revendications 1 à 7, dans lequel
(a) le ou les agents liants est ou sont dissous ou mis en suspension dans de l'eau purifiée,
(b) la terre médicamenteuse est mélangée au mélange d'agent liant et d'eau selon (a) et est granulée selon un procédé usuel, et
(c) le ou les polymères ou agents filmogènes hydrosolubles du revêtement est ou sont dissous ou mis en suspension dans de l'eau purifiée et
(d) le granulé obtenu selon (b) est revêtu du mélange de revêtement et d'eau selon (c) par un procédé usuel.

9. Procédé selon la revendication 8,
dans lequel l'étape de procédé (b) est réalisée avec une extrudeuse.

10. Procédé selon la revendication 8 ou 9, dans lequel l'étape de procédé selon (b) et/ou l'étape de procédé selon (d) est ou sont réalisés dans un lit fluidisé.

11. Procédé selon la revendication 10,
dans lequel l'étape de procédé selon (b) et/ou l'étape de procédé selon (d) est ou sont réalisées assez longtemps pour que l'épaisseur de revêtement souhaitée et l'humidité résiduaire requise soient atteintes.
